# EUROPEAN PATENT APPLICATION

(11) **EP 4 299 048 A1**
(43) Date of publication of application: **03.01.2024**
(21) Application number: 22182432.9
(22) Date of filing: 30.06.2022
(51) Int. Cl.: A61K 6/35, A61K 6/884, C08L 1/28, C08L 35/08

(54) **DENTURE ADHESIVE**

(71) Applicant: GlaxoSmithKline Consumer Healthcare (UK) IP Limited, Brentford, Middlesex TW8 9GS (GB)
(72) Inventor: The designation of the inventor has not yet been filed
(74) Representative: Haleon Patent Department

(57) **Abstract**

The present invention relates to denture adhesive compositions. The compositions comprise (a) carboxymethylcellulose, (b) a cross-linked polyacrylic acid homopolymer and (c) an alkyl vinyl ether-maleic acid copolymer or salt thereof. Advantageously, the compositions provide an immediate, strong and long-lasting hold.

## Description

### FIELD OF THE INVENTION

The present invention relates to denture adhesive compositions. The compositions comprise (a) carboxymethylcellulose, (b) a cross-linked polyacrylic acid homopolymer and (c) an alkyl vinyl ether-maleic acid copolymer or salt thereof. Advantageously, the compositions provide an immediate, strong and long-lasting hold.

### BACKGROUND TO THE INVENTION

Full or partial dentures are forms of dental appliance intended to be worn in the mouth as substitutes serving as a replacement for some or all of the teeth usually found in the oral cavity. When using dentures, it is common to use an adhesive to help adhere the dental appliance to a surface of the oral cavity, such as the palate or a gum ridge. Such denture adhesives secure or temporarily fix dentures within the mouth, improving retention of the dentures, whilst also being releasable so that the denture wearer may remove the dentures for cleaning and maintenance. It is also recommended to remove dentures at the end of the day. Denture adhesives can provide an improvement in the denture wearing experience even if the denture fits well. Denture adhesives are also beneficial in situations where dentures might not fit perfectly, for example as a consequence of natural shrinkage and changes in the gum or oral mucosa over time.

Denture adhesive compositions, (also known as fixative compositions) fill the interstices between the dentures and the gums or tissues. Prior to placement of the denture in the oral cavity, the denture adhesive is applied to the denture plate surface to provide uniform contact with the gums and oral tissues.

Denture adhesive compositions need to fulfill a number of, sometimes competing, criteria in order to function effectively: they should develop a high degree of tack upon contact with saliva so that the dentures are held in place as soon as they are seated in the mouth, possess sufficient cohesive strength to withstand the stresses of mastication which act to dislodge the denture and provide a seal against ingress of food residue beneath the denture. Further, the organoleptic properties of the denture adhesive composition, such as mouthfeel and consistency should be acceptable to the user and the composition should not be too messy or sticky to apply.

Denture adhesives are generally sold as a cream, liner or strip, liquid, gel or powder, and examples are known in the art including commercially available denture adhesives such as POLIGRIP Flavour Free denture adhesive cream (GlaxoSmithKline), POLIGRIP Cushion and Comfort denture adhesive cream (GlaxoSmithKline) and Fixodent PLUS Best Hold Premium denture adhesive cream (P&G).

WO/1992/22280 (Richardson Vicks, Inc.) discloses denture stabilizing compositions comprising mixed partial salts of lower alkyl vinyl ether-maleic acid copolymers.

WO/2013/054236 (Procter and Gamble) discloses denture adhesives comprising a mixture of a partial salt of a copolymer of methyl vinyl ether-maleic acid, sodium carboxy methyl cellulose and a cohesion builder component in an amount from about 0.001% to about 10% by weight of the denture adhesive composition.

WO/2016/091738 (Glaxo Group Ltd) discloses denture adhesive compositions comprising mixed partial salts of lower alkyl vinyl ether-maleic acid copolymers, sodium carboxy methyl cellulose and a bioactive glass.

WO/2018/175826 (Lubrizol) discloses compositions that include (a) a cross linked poly(acrylic) acid polymer, in combination with (b) a carbomethylcellulose (CMC) component and/or (c) an adherence promoting component. The cross-linked poly(acrylic) acid polymer may be a carbomer homopolymer, carbomer copolymer, carbomer interpolymer, polycarbophil or a mixture thereof.

Despite considerable effort to develop denture adhesive compositions over the years, there remains a need for further denture adhesives offering improved user experience.

### SUMMARY OF THE INVENTION

The Inventors have developed compositions with desirable adhesive properties, particularly for use with dental appliances such as dentures.

In a First Aspect, there is provided a denture adhesive composition comprising: (a) carboxymethylcellulose, (b) a cross-linked polyacrylic acid homopolymer and (c) an alkyl vinyl ether-maleic acid copolymer or salt thereof.

Particularly, the denture adhesive composition comprises: (a) from about 15 to about 30 percent by weight (%w/w) of carboxymethylcellulose, (b) from about 6 to about 15 percent by weight (%w/w) of the cross-linked polyacrylic acid homopolymer and (c) from about 15 to about 34 percent by weight (%w/w) of an alkyl vinyl ether-maleic acid copolymer or salt thereof.

Denture adhesive compositions of the Invention may further comprise (d) an oil component and/or (e) a petrolatum component. Particularly, denture adhesive compositions of the Invention may comprise from about 17 to about 56 percent by weight (%w/w) of the oil component, petrolatum component or combination thereof.

In some embodiments, the denture adhesive composition of the Invention may comprise from about 17 to about 56 percent by weight (%w/w) of an oil component. In some embodiments, the denture adhesive composition of the Invention may comprise from about 17 to about 56 percent by weight (%w/w) of a petrolatum component. Particularly, the denture adhesive composition of the Invention may comprise from about 17 to about 26 percent by weight (%w/w) of an oil component and from about 20 to about 30 percent by weight (%w/w) of a petrolatum component.

Particularly, following preparation, the denture adhesive compositions of the Invention have a viscosity of at least 500,000 centipoise (cP). Particularly, following preparation, the denture adhesive compositions of the Invention have a viscosity of no more than 2,500,000 cP, more particularly no more than 2,000,000 cP. Particularly, following preparation, the denture adhesive compositions of the Invention have a viscosity of from 500,000 to 2,500,000 centipoise (cP), more particularly of from 500,000 to 2,000,000 cP. Particularly the compositions have a pH of from 4.0 to 10, more particularly a pH of from 5.0 to 8.0. More particularly the denture adhesive compositions of the Invention have (i) a viscosity of from 500,000 to 2,000,000 centipoise (cP) and/or (ii) a pH of from 4.0 to 10, more particularly a pH of from 5.0 to 8.0.

Particularly, the carboxymethylcellulose has a viscosity of about 1000 to about 2800 centipoise (cP) (measured in a 1% solution at 25°C). More particularly, the carboxymethylcellulose consists of fine particles of which at least 80% pass through a 200 U.S. mesh sieve. Preferably, the carboxymethylcellulose is sodium carboxymethylcellulose.

Particularly, the denture adhesive compositions of the Invention comprise from about 5 to about 10 percent by weight (%w/w) of the cross-linked polyacrylic acid homopolymer, particularly from about 6 to about 9 percent by weight (%w/w) of the cross-linked polyacrylic acid homopolymer.

Particularly, the cross-linked polyacrylic acid homopolymer is an allyl pentaerythritol crosslinked homopolymer. More particularly, the cross-linked polyacrylic acid homopolymer is a carbomer homopolymer Type A. Yet more particularly, the cross-linked polyacrylic acid homopolymer is selected from the group consisting of Carbopol 971P NF, Carbopol 974P NF and combinations thereof. Preferably, the cross-linked polyacrylic acid homopolymer is Carbopol 971P NF.

Particularly, the alkyl vinyl ether-maleic acid copolymer or salt thereof is a poly(methylvinylether/maleic acid) salt. More particularly, the alkyl vinyl ether-maleic acid copolymer or salt thereof is a poly(methylvinylether/maleic acid) Sodium-Calcium Mixed Partial Salt.

In certain embodiments, the denture adhesive composition comprises or consists essentially of:
(a) from 26-30% w/w of sodium carboxymethylcellulose;
(b) from 6-10% w/w of a cross-linked polyacrylic acid homopolymer;
(c) from 16-26% w/w of poly(methylvinylether/maleic acid) Sodium-Calcium Mixed Partial Salt;
(d) from 17-22% w/w of an oil component;
(e) from 23%-29% of petrolatum; and optionally
(f) at least one fragrance, colorant, preservative, surfactant or mixture thereof;
wherein the composition has a viscosity of from 1,000,000 to 2,000,000 centipoise (cP) and a pH of from 5.0 to 7.5.

Particularly, denture adhesive compositions do not contain polyethylene glycol, sodium alginate and/or zinc. Preferably, denture adhesive compositions of the Invention are free of polyethylene glycol, sodium alginate and zinc.

In a Second Aspect, there is provided a denture adhesive composition according to the First Aspect for use in a method of adhering a dental appliance to a surface, particularly a surface in the oral cavity. There is also provided a method of adhering a dental appliance to a surface, particularly a surface in the oral cavity, using a denture adhesive composition according to the First Aspect. Particularly, the methods may comprise applying a composition of the First Aspect to a dental appliance, the oral cavity, or both, and applying the dental appliance to a surface of the oral cavity, such as the palate or a gum ridge.

### DESCRIPTION OF FIGURES

**FIGURE 1****:** Shows adhesion profiles of formulations 1-6 in comparison to a control formulation (POLIGRIP Flavour Free denture adhesive cream).
**FIGURE 2****:** Shows adhesion profiles of formulations 7 to 14 in comparison to a control formulation (POLIGRIP Flavour Free denture adhesive cream).
**Figure 3****:** Shows adhesion profiles for formulations 16, 17 and 20 in comparison to a control formulation (POLIGRIP Flavour Free denture adhesive cream).
**FIGURE 4****:** Shows the effect of changing the proportion of mineral oil and petrolatum on adhesion profiles for formulations 22, 23 and 24.
**Figure 5****:** Shows the adhesive layer thickness formulations 22, 23 and 24 before and during compression. Each of the formulations maintained a higher layer thickness than the control (Figure 5(a).

### DETAILED DESCRIPTION OF THE INVENTION

The present inventors have discovered improved denture adhesive compositions that address a number of concerns with currently available denture adhesives. The compositions provide an immediate hold, helping to make dentures feel natural and comfortable in the mouth, avoiding concerns with traditional denture adhesives that may take time to develop good hold after initial application. Furthermore, the denture adhesive formulations offer a strong and long-lasting hold. The materials adhere to the gum ridge and, in addition to providing adhesive properties, create a cushioning effect between the gums and the denture, improving overall comfort. The denture adhesive formulations may also reduce problems with oozing that can occur where adhesive leaks out between the gum line and denture into the oral cavity.

Denture adhesive compositions of the Invention comprise: (a) carboxymethylcellulose, (b) a crosslinked polyacrylic acid homopolymer and (c) an alkyl vinyl ether-maleic acid copolymer or salt thereof, wherein the composition has a pH of from 5.0 to 8.0, particularly a pH of from 5.0 to 7.5.

As used herein, the term "dental appliance" refers to dentures or partial dentures, artificial teeth, removable orthodontic bridges and denture plates, both upper and lower types, orthodontic retainers and appliances, protective mouthguards, nightguards to prevent bruxism and/or Temporomandibular joint (TMJ) disorder, and the like. The term "denture" is used to refer to a subset of dental appliances which includes dentures or partial dentures, such as upper and lower dentures and combinations thereof, including orthodontic bridges, artificial teeth, denture plates and the like.

The terms "denture adhesive composition" and "denture adhesive" are terms used in the art and refer to a composition for use with a denture to enhance retention, stability and function.

For the avoidance of doubt, reference to "% by weight" (%w/w) means by weight of the total composition, the amount of each ingredient being selected so that total ingredients in the composition sum to 100 percent by weight.

### Carboxymethylcellulose

Denture adhesive compositions of the Invention comprise carboxymethylcellulose (CMC), a cellulose derivative. Particularly, the compositions comprise from about 15% to about 30% by weight of CMC, particularly from about 20% to about 30% by weight or more particularly, from about 26% to about 30% by weight of CMC. For example, 25%, 26%, 27%, 28%, 29% or 30% by weight of CMC. Preferably the CMC is food or pharmaceutical grade CMC.

Particularly, the CMC is provided in the form of a powder prior to use in a composition of the Invention, more particularly in the form of a fine powder, more particularly in the form of a fine powder of which at least 99.9% of the particles pass through a 60 U.S. mesh sieve, more particularly at least 90% of the particles pass through an 80 U.S. mesh sieve, yet more particularly at least 80% of the particles pass through a 100 U.S. mesh sieve and still yet more particularly at least 80% of the particles pass through a 200 U.S. mesh sieve

Particularly, the CMC has a viscosity of from about 800 to about 3000 centipoise (cP), preferably a viscosity of from about 1000 to about 2800 cP (measured in a 1% solution at 25°C).

Particularly, the CMC has a degree of substitution (DS) of from about 0.65 to about 0.95, such as from about 0.65 to about 0.85, preferably about 0.7.

Preferably the CMC is sodium carboxymethylcellulose. Particularly the sodium carboxymethylcellulose having a sodium content of from 6.5% to 9.5% such as from 7.0% to about 9.2%, more particularly of from about 7.0% to about 8.9%.

Preferably the CMC is sodium carboxymethylcellulose having an average molecular weight of about 725,000.

Examples of commercially available CMCs useful in compositions of the Invention include the 7H products available from Aqualon such as 7H3SXF or 7H3SF and products available from Dupont such as WALOCEL CRT 2000, WALOCEL CRT 10000 or WALOCEL CRT 15000, such as WALOCEL CRT 15000PPA (S) Sodium. In certain embodiments, the sodium CMC is sodium CMC 7H3SXF, 7H3SF, 7H3SXF PH, 7H3SF PH, WALOCEL CRT 15000, WALOCEL CRT 15000PPA (S) Sodium or equivalents.

In certain embodiments, the sodium carboxymethylcellulose is food or pharmaceutical grade CMC having a viscosity of from about 1000 to about 2800 cP (measured in a 1% solution at 25°C), a degree of substitution of from about 0.65 to about 0.95, a sodium content of from 6.5% to 9.5% and an average molecular weight of about 725,000.

### Cross-linked polvacrvlic acid homopolymer

Denture adhesive compositions of the Invention comprise a cross-linked polyacrylic acid homopolymer, generally referred to using the generic term "carbomer". Carbomers are high weight polymers of acrylic acid cross-linked with either allyl sucrose or allyl ethers of pentaerythritol containing 56%-68% of carboxylic acid (-COOH) groups and 0.75%-2% of cross-linking agents. On contact with water, carbomers absorb water readily, swelling as they become hydrated.

Particularly, the compositions comprise from about 4% to about 15% by weight of the cross-linked polyacrylic acid homopolymer, particularly from about 5% to about 10% by weight or more particularly, from about 6% to about 9% by weight of the cross-linked polyacrylic acid homopolymer. For example, 4%, 5%, 6%, 7%, 8%, or 9% by weight of the cross-linked polyacrylic acid homopolymer.

Particularly, the cross-linked polyacrylic acid homopolymer is an allyl pentaerythritol crosslinked homopolymer. More particularly, the cross-linked polyacrylic acid homopolymer is an allyl pentaerythritol crosslinked homopolymer polymerized in ethyl acetate. The cross-linked polyacrylic acid homopolymer may be a carbomer homopolymer Type A or Type B. In some embodiments, the cross-linked polyacrylic acid homopolymer is a carbomer homopolymer Type A. In some embodiments, the cross-linked polyacrylic acid homopolymer is a carbomer homopolymer Type B.

Particularly, the cross-linked polyacrylic acid homopolymer is food and/or pharmaceutical grade and provided in the form of a free-flowing powder prior to use in a composition of the Invention, more particularly in the form of a fine free-flowing powder.

In certain embodiments, the CAS number for the cross-linked polyacrylic homopolymer is 9003-01-4. Preferably, the cross-linked polyacrylic acid homopolymer is selected from the group consisting of carbomer 971, carbomer 974 and combinations thereof. More preferably, the cross-linked polyacrylic acid homopolymer is carbomer 971 or carbomer 974. In preferred embodiments, the cross-linked polyacrylic acid homopolymer is carbomer 971.

Suitable commercially available forms of carbomer 971 or carbomer 974 are available from LUBRIZOL, for example as CARBOPOL 971P NF and CARBOPOL 974P NF, or from COREL PHARMA, for example as ACRYPOL 971 and ACRYPOL 974. In particular embodiments, the cross-linked polyacrylic homopolymer is CARBOPOL 971P NF.

### Alkyl vinyl ether-maleic acid copolymer

The denture adhesive compositions of the Invention comprise an alkyl vinyl ether-maleic acid (AVE/MA) copolymer or salt thereof, such as a mixed salt, a partial salt or a mixed partial salt. As used herein, the term "mixed salt" refers to salts wherein at least two different cations are mixed on the same polymer with each other or with other salts. The term, "partial salt" refers to salts of polymers where less than 100 percent of the acid groups are reacted.

Particularly, the denture adhesive compositions comprise an alkyl vinyl ether-maleic acid copolymer partial salt. More particularly, the denture adhesive compositions comprise an alkyl vinyl ether-maleic acid copolymer mixed partial salt.

The alkyl vinyl ether-maleic acid copolymer comprises at least one cationic salt selected from the group consisting of strontium, zinc, iron, magnesium, calcium and sodium. Preferably, the alkyl vinyl ether-maleic acid copolymer contains at least one cationic salt selected from the group consisting of calcium and sodium. Thus, in some embodiments the AVE/MA copolymer contains a cationic salt selected from calcium and sodium or a combination thereof. Preferably, the at least one cationic salt is a combination of calcium and sodium cations.

The metal cations may be present on the alkyl vinyl ether-maleic acid copolymer in an amount to provide from about 10.0 percent to about 20.0 percent of metal cations, such as about 12.0% to about 16.0%, for example about 12.0%, about 12.5%, about 13.0%, about 13.5%, about 14.0%, about 14.5%, about 15.0% or about 15.5%.

Particularly, the alkyl vinyl ether-maleic acid copolymer comprises or consists essentially of from about 1.0 percent to about 15.0 percent calcium cations and from about 0.5 percent to about 5.0 percent sodium cations. More particularly, the alkyl vinyl ether-maleic acid copolymer comprises or consists essentially of from about 9.0 percent to about 15.0 percent calcium cations and from about 1.0 percent to about 3.0 percent sodium cations. Preferably the alkyl vinyl ether-maleic acid copolymer is free of zinc cations.

Suitably, the alkyl vinyl ether-maleic acid copolymer will have a molecular weight of at least 900,000 Daltons. In certain embodiments the molecular weight is from about 1,000,000 to about 3,000,000.

Particularly, the denture adhesive composition comprises from about 15 to about 34 percent by weight of the alkyl vinyl ether-maleic acid copolymer or salt thereof. More particularly, the denture adhesive composition comprises from about 15 to about 26 percent by weight of the alkyl vinyl ether-maleic acid copolymer or salt thereof. Yet more particularly, the denture adhesive composition comprises from about 15 to about 22 percent by weight of the alkyl vinyl ether-maleic acid copolymer or salt thereof. Still yet more particularly, the denture adhesive composition comprises from about 16 to about 20 percent by weight of the alkyl vinyl ether-maleic acid copolymer or salt thereof, such as about 16 percent by weight, about 17 percent by weight, about 18 percent by weight, about 19 percent by weight or about 20 percent by weight.

Preferably, the alkyl vinyl ether-maleic acid copolymer or salt thereof is a poly(methylvinylether/maleic acid) salt. More preferably, the alkyl vinyl ether-maleic acid copolymer or salt thereof is a poly(methylvinylether/maleic acid) Sodium-Calcium Mixed Partial Salt.

Examples of suitable commercially available alkyl vinyl ether-maleic acid copolymers are sold under the trade name GANTREZ (ISP Investments LLC) available from Thermo Fisher or Ashland. These include the GANTREZ AN (Anhydride) form such as GANTREZ AN169 (a copolymer of methyl vinyl ether/maleic anhydride) and the GANTREZ MS form such as GANTREZ MS- 955 (a mixed sodium and calcium salt of methyl vinyl ether and maleic anhydride copolymer).

### Other Components

Denture adhesive compositions of the Invention may further comprise (d) an oil component and/or (e) a petrolatum component.

The oils useful in compositions of the Invention may have a kinematic viscosity of from about 10 centistokes (cSt, mm²/s) up to about 240 cSt at 40°C, more particularly from about 10 cSt to about 100 Cst at 40°C or yet more particularly from about 10 cSt to about 90 cSt at 40°C. Kinematic viscosity may be measured at 40°C by means of a calibrated glass capillary viscometer as set out in the ASTM Standard Test Method for Kinematic Viscosity of Transparent and Opaque Liquids D445 (DOI: 10.1520/D0445-21E01).

Suitable oils for use in compositions of the Invention include, by way of non-limiting example, light mineral oil, medium mineral oil, vegetable oils such as olive oil, oleic acid, sesame oil, safflower oil, corn oil, soybean oil, cottonseed oil, castor oil, palm oil and coconut oil.

Preferably the oil component is selected from the group consisting of light mineral oil, medium mineral oil and combinations thereof.

Mineral oils may be characterised in terms of their kinematic viscosity. A light mineral oil will generally have a kinematic viscosity of about 34 cSt or less at 40°C. Medium and heavy mineral oils will generally have a kinematic viscosity ranging from about 35 cSt up to about 240 cSt at 40°C.

In certain embodiments, the oil component is medium mineral oil. In certain embodiments, the oil is a medium mineral oil, more particularly a medium mineral oil having a kinematic velocity of from about 35 cSt to about 90 cSt at 40°C and yet more particularly a medium mineral oil having a kinematic velocity of from about 63 cSt to about 80 cSt at 40°C. Suitable medium mineral oils include PIONIER 2071P or equivalent.

In certain embodiments, the oil component is light mineral oil. In certain embodiments, the oil is a light mineral oil, more particularly a light mineral oil having a kinematic velocity of from about 10 cSt to about 34 cSt at 40°C, yet more particularly a medium mineral oil having a kinematic velocity of from about 10 cSt to about 20 cSt at 40°C, still yet more particularly a medium mineral oil having a kinematic velocity of from about 14 cSt to about 17 cSt at 40°C. Suitable light mineral oils include BLANDOL white mineral oil or equivalent.

Medium mineral oils (as defined above) are preferred for use in the invention.

The term "petrolatum" is a term of the art used to refer to a semi-solid mixture of hydrocarbons. The viscosity of petrolatum may be determined using the ASTM Standard Test Method for Cone Penetration of Petrolatum (D937-07) to determine the penetration of petrolatum as an empirical measure of consistency. The petrolatum may have a cone penetration consistency value (mm/10 at 25°C) of from about 10 to about 500, particularly from about 25 to about 300 and more particularly from about 50 to about 250. Preferably, the petrolatum has a cone penetration consistency value of from about 100 to about 200, more preferably from about 130 to about 195 (mm/10 at 25°C).

Particularly, denture adhesive compositions of the Invention may comprise from about 10 to about 56 percent by weight (%w/w) of the oil component and/or petrolatum component, more particularly from about 17 to about 56 percent by weight (%w/w) of the oil component and/or petrolatum component.

In some embodiments, the denture adhesive composition of the Invention may comprise from about 17 to about 56 percent by weight (%w/w) of an oil component. In some embodiments, the denture adhesive composition of the Invention may comprise from about 17 to about 56 percent by weight (%w/w) of a petrolatum component.

Particularly, the denture adhesive composition of the Invention may comprise from about 17 to about 26 percent by weight (%w/w) of an oil component and from about 20 to about 30 percent by weight (%w/w) of a petrolatum component. More particularly, the denture adhesive composition of the Invention may comprise from about 18 to about 22 percent by weight (%w/w) of an oil component and from about 20 to about 27 percent by weight (%w/w) of a petrolatum component. Yet more particularly, the denture adhesive composition of the Invention may comprise from about 18 to about 22 percent by weight (%w/w) of an oil component and from about 23 to about 27 percent by weight (%w/w) of a petrolatum component. Still yet more particularly, the denture adhesive composition of the Invention may comprise from about 18 to about 22 percent by weight (%w/w) of an oil component, such as about 18%, about 19%, about 20%, about 21% or about 22%, and from about 23 to about 27 percent by weight (%w/w) of a petrolatum component, such as about 23%, about 24%, about 25%, about 26% or about 27%. In preferred embodiments, the denture adhesive composition of the Invention comprises about 18 percent by weight (%w/w) of an oil component and about 25 percent by weight (%w/w) of a petrolatum component. In further preferred embodiments, the denture adhesive composition of the Invention comprises about 18 percent by weight (%w/w) of a medium mineral oil component and about 25 percent by weight (%w/w) of a petrolatum component.

### Sensory Components

Denture adhesive compositions may comprise at least one sensory component. A "sensory component" refers to a component that may be used to impart a flavour, fragrance, colour and/or sensation (such as cooling, warming or tingling) to the composition. Other than imparting the compositions with organoleptic properties that are desirable for consumers, generally the sensory component is not an active component, in other words it does not play an active role in adhesion, hold time or cushioning. Particularly, the sensory component is at least one flavour, fragrance and/or colouring agent. Sensory components are known in the art and may be natural or artificial (synthetic).

Compositions of the invention may comprise from about 0.01% to about 5% by weight of a sensory component, particularly from about 0.01% to about 2% by weight of a sensory component.

Flavouring agents well known in the denture adhesive art may be added to the compositions of the present invention. These flavouring agents include, but are not limited to, synthetic flavour oils and/or oils derived from plants, leaves, flowers, fruits and so forth, and combinations thereof. Representative flavour oils include spearmint oil, cinnamon oil, oil of wintergreen (methylsalicylate) and peppermint oils. Also useful are artificial, natural or synthetic fruit flavours such as citrus oil including lemon, orange, grape, lime, and grapefruit, and fruit essences including apple, strawberry, cherry, pineapple, and so forth. The flavouring agent may be a liquid, spray dried, encapsulated, or absorbed on a carrier, and mixtures thereof. In one aspect, the flavouring agent is peppermint oil. The amount of flavouring agent utilized varies depending on such factors as flavour type, adhesive composition and strength desired. In one aspect, the denture adhesive composition comprises from about 0.01 percent to about 3.0 percent by weight of the flavouring agent. In another aspect, the denture adhesive composition comprises from about 0.01 percent to about 2.0 percent by weight of the flavouring agent. In another aspect, the denture adhesive composition comprises from about 0.05 percent to 2.0 percent by weight of the flavouring agent.

The denture adhesive compositions may also include the use of sweeteners well known in the art. The sweetening agent may be selected from a wide range of materials including water-soluble agents, water-soluble artificial sweeteners, and dipeptide based sweeteners, including mixtures thereof. Representative sweeteners include, but are not limited to, (a) sugar alcohols such as sorbitol, xylitol, mannitol, maltitol, hydrogenated starch hydrolysate, and mixtures thereof; (b) water-soluble artificial sweeteners such as the soluble saccharin salts, i.e., sodium or calcium saccharin salts, cyclamate salts, Acesulfame-K, sucralose, and the like, and the free acid form of saccharin; (c) dipeptide based sweeteners such as L-aspartyl-L-phenylalanine methyl ester, and the like; and (d) natural sweeteners such as stevia. In one aspect, the denture adhesive composition comprises from about 0.001 percent to about 5.0 percent by weight of the sweetening agent.

The colourants useful in the present invention include the pigments and dyes suitable for food, drug and cosmetic applications. These colourants are known as FD&C (Food, Drugs and Cosmetics) and D&C (Drugs & Cosmetics) dyes and lakes. Illustrative examples include, but are not limited to, D&C red#30 Aluminium Lake Paste, D&C Red #7 Calcium Lake Paste, Erythrosine Lake Paste, indigo dye, known as FD&C Blue No. 2, which is the disodium salt of 5,5 -indigotindi-sulfonic acid; FD&C Green No. 1, comprising a triphenylmethylene dye and is the monosodium salt of the 4-[4-N- ethyl-p-sulfobenzylamino) diphenylmethylene]-[I-(N-ethyl-N-P-sulfobenzyl)-2,5- cyclohexadienimine]. In some embodiments, the colorant is FD&C Red No. 3. In some embodiments, the denture adhesive composition comprises from about 0 percent to about 0.2 percent by weight of colourant.

In preferred embodiments, the denture adhesive composition is free of flavour, fragrance and/or colouring agents.

Denture adhesive compositions of the Invention may be dispensed by the consumer from a multidose package, pump or tube. Alternatively, the denture adhesive compositions of the Invention may be pre-dispensed, in a single dose packages, for example on a non-adhesive self-supporting layer that may be peeled off to enable the adhesive to be applied.

To facilitate dispensing, when measured following preparation of the composition, the denture adhesive compositions of the Invention may have a viscosity of from 500,000-2,500,000 centipoise (cP), more particularly a viscosity of from 1,000,000 to 2,000,000 cP. For the avoidance of doubt, viscosity may be measured within two months of preparation of the composition, more particularly within one month of preparation of the composition, such as a viscosity of from 1,000,000 to 2,000,000 cP when measured within one month of preparation of the composition.

Compositions of the invention preferably have a pH within the range of from 4.0 to 10.0, such as a pH within the range of from 5.0 to 8.0, preferably a pH of from about 5.0 to about 7.5, such as a pH of from about 5.2 to about 7.1.

Typically, compositions of the invention are stable for at least 3 months at a temperature of 25°C, particularly for at least 6 months at a temperature of 25°C, more particularly for at least 12 months at a temperature of 25°C, yet more particularly for at least 24 months at a temperature of 25°C and still yet more particularly for 36 months at a temperature of 25°C. As used herein, the term "stable" refers to the ability of the composition to maintain certain physical or chemical properties, such as adhesion, at a specific storage temperature for a period of time after production. The stability of compositions of the present invention may be assessed using standard methods known in the art.

Particularly, the proportion of (a) carboxymethylcellulose, (b) cross-linked polyacrylic acid homopolymer and (c) alkyl vinyl ether-maleic acid copolymer or salt thereof in the denture adhesive composition is 54% w/w (i.e. (a)+(b)+(c)=54% w/w).

Particularly, denture adhesive compositions do not contain polyethylene glycol, sodium alginate and/or zinc. Preferably, denture adhesive compositions of the Invention are free of polyethylene glycol, sodium alginate and zinc. Particularly, denture adhesive compositions of the Invention do not comprise SiO₂.

There is provided a denture adhesive composition of the Invention for use in a method of adhering a dental appliance to a surface, particularly a surface in the oral cavity. There is also provided a method of adhering a dental appliance to a surface, particularly a surface in the oral cavity, using a denture adhesive composition of the Invention.

Particularly, the methods may comprise applying a composition of the First Aspect to a dental appliance, the oral cavity, or both, and applying the dental appliance to a surface of the oral cavity, such as the palate or a gum ridge.

### General

The term "comprising" encompasses "including" e.g. a composition "comprising" X may include something additional e.g. X + Y. In some implementations, the term "comprising" refers to the inclusion of the indicated active agent, such as recited compounds, as well as inclusion of other active agents, and carriers, excipients, emollients, stabilizers, etc., known in the consumer health industry and generally recognized as safe (GRAS). Use of the transitional phrase "consisting essentially of" means that the scope of a claim is to be interpreted to encompass the specified materials or steps recited in the claim, and those that do not materially affect the basic and novel characteristic(s) of the claimed invention. See, In re Herz, 537 F.2d 549, 551-52, 190 USPQ 461, 463 (CCPA 1976) (emphasis in the original); see also MPEP § 2111.03. Thus, the term "consisting essentially of" when used in a claim of this invention is not intended to be interpreted to be equivalent to "comprising". The term "consisting of" and variations thereof means including and limited to (for example, the specific recited constituents or steps). In certain territories, the term "comprising an active ingredient consisting of" may be used in place of "consisting essentially". The term "about" in relation to a numerical value x is optional and means, for example, that the numerical value may comprise some variance around the stated number to allow for routine experimental fluctuation, measurement variance or to encompass minor deviations that may achieve substantially the same results as the stated number, such as x±10%, x±5%, x±4%, x±3%, x±2% or x±1%. The word "substantially" does not exclude "completely" e.g. a composition which is "substantially free" from Y may be completely free from Y. Where necessary, the word "substantially" may be omitted from the definition of the invention. All percentages and ratios used herein are by weight of total composition, unless otherwise indicated.

All references or patent applications cited within this patent specification are incorporated by reference herein.

### Embodiments of the Invention

The following clauses describe certain embodiments of the invention:
**Embodiment 1:** A denture adhesive composition comprising or consisting essentially of:
   (a) from 26-30% w/w of sodium carboxymethylcellulose;
   (b) from 6-9% w/w of a cross-linked polyacrylic acid homopolymer;
   (c) from 16-20% w/w of poly(methylvinylether/maleic acid) Sodium-Calcium Mixed Partial Salt;
   (d) from 17-22% w/w of an oil component;
   (e) from 23%-29% of petrolatum; and optionally
   (f) at least one fragrance, colorant, preservative, surfactant or mixture thereof.
**Embodiment 2:** A denture adhesive composition comprising or consisting essentially of:
   (a) from 26-30% w/w of sodium carboxymethylcellulose;
   (b) from 6-9% w/w of a cross-linked polyacrylic acid homopolymer;
   (c) from 16-20% w/w of poly(methylvinylether/maleic acid) Sodium-Calcium Mixed Partial Salt;
   (d) from 17-22% w/w of an oil component;
   (e) from 23%-29% of petrolatum; and optionally
   (f) at least one fragrance, colorant, preservative, surfactant or mixture thereof;
   wherein the composition has a viscosity of from 1,000,000 to 2,000,000 centipoise (cP) and a pH of from 5.0 to 7.5.
**Embodiment 3:** A denture adhesive composition comprising or consisting essentially of:
   (a) from 26-30% w/w of sodium carboxymethylcellulose;
   (b) from 6-9% w/w of a cross-linked polyacrylic acid homopolymer;
   (c) from 16-20% w/w of poly(methylvinylether/maleic acid) Sodium-Calcium Mixed Partial Salt;
   (d) from 18-22% w/w of an oil component; and
   (e) from 23%-27% of petrolatum.
**Embodiment 4:** A denture adhesive composition comprising or consisting essentially of:
   (a) from 26-30% w/w of sodium carboxymethylcellulose;
   (b) from 6-9% w/w of a cross-linked polyacrylic acid homopolymer;
   (c) from 16-20% w/w of poly(methylvinylether/maleic acid) Sodium-Calcium Mixed Partial Salt;
   (d) from 18-22% w/w of an oil component;
   (e) from 23%-27% of petrolatum;
   wherein the composition has a viscosity of from 1,000,000 to 2,000,000 centipoise (cP) and a pH of from 5.0 to 7.5.
**Embodiment 5:** A denture adhesive composition comprising or consisting essentially of:
   (a) from 26-30% w/w of sodium carboxymethylcellulose;
   (b) about 8% w/w of a cross-linked polyacrylic acid homopolymer;
   (c) about 18% w/w of poly(methylvinylether/maleic acid) Sodium-Calcium Mixed Partial Salt;
   (d) from 17-22% w/w of an oil component;
   (e) from 23%-29% of petrolatum;
   wherein the composition has a viscosity of from 1,000,000 to 2,000,000 centipoise (cP) and a pH of from 5.0 to 7.5.
**Embodiment 6:** A denture adhesive composition comprising, consisting or consisting essentially of:
   (a) about 28% w/w of sodium carboxymethylcellulose;
   (b) about 6% w/w of a cross-linked polyacrylic acid homopolymer;
   (c) about 20% w/w of poly(methylvinylether/maleic acid) Sodium-Calcium Mixed Partial Salt;
   (d) from 17-22% w/w of an oil component;
   (e) from 23%-29% of petrolatum;
   wherein the composition has a viscosity of from 1,000,000 to 2,000,000 centipoise (cP) and a pH of from 5.0 to 7.5.
**Embodiment 7:** A denture adhesive composition comprising, consisting or consisting essentially of:
   (a) about 28% w/w of sodium carboxymethylcellulose;
   (b) about 6% w/w of a cross-linked polyacrylic acid homopolymer;
   (c) about 20% w/w of poly(methylvinylether/maleic acid) Sodium-Calcium Mixed Partial Salt;
   (d) about 17% w/w of an oil component;
   (e) about 29% of petrolatum;
   wherein the composition has a viscosity of from 1,000,000 to 2,000,000 centipoise (cP) and a pH of from 5.0 to 7.5.
**Embodiment 8:** A denture adhesive composition comprising or consisting essentially of:
   (a) about 28% w/w of sodium carboxymethylcellulose;
   (b) about 6% w/w of a cross-linked polyacrylic acid homopolymer;
   (c) about 20% w/w of poly(methylvinylether/maleic acid) Sodium-Calcium Mixed Partial Salt;
   (d) about 21% w/w of an oil component;
   (e) about 25% of petrolatum;
**Embodiment 9:** A denture adhesive composition comprising, consisting essentially or consisting of:
   (a) about 28% w/w of sodium carboxymethylcellulose;
   (b) about 8% w/w of a cross-linked polyacrylic acid homopolymer;
   (c) about 18% w/w of poly(methylvinylether/maleic acid) Sodium-Calcium Mixed Partial Salt;
   (d) from 17-22% w/w of an oil component;
   (e) from 23%-29% of petrolatum;
   wherein the composition has a viscosity of from 1,000,000 to 2,000,000 centipoise (cP) and a pH of from 5.0 to 7.5.
**Embodiment 10:** A denture adhesive composition comprising, consisting or consisting essentially of:
   (a) about 28% w/w of sodium carboxymethylcellulose;
   (b) about 8% w/w of a cross-linked polyacrylic acid homopolymer;
   (c) about 18% w/w of poly(methylvinylether/maleic acid) Sodium-Calcium Mixed Partial Salt;
   (d) about 17% w/w of an oil component;
   (e) about 29% of petrolatum;
   wherein the composition has a viscosity of from 1,000,000 to 2,000,000 centipoise (cP) and a pH of from 5.0 to 7.5.
**Embodiment 11:** A denture adhesive composition comprising or consisting essentially of:
   (a) about 28% w/w of sodium carboxymethylcellulose;
   (b) about 8% w/w of a cross-linked polyacrylic acid homopolymer;
   (c) about 18% w/w of poly(methylvinylether/maleic acid) Sodium-Calcium Mixed Partial Salt;
   (d) about 21% w/w of an oil component;
   (e) about 25% of petrolatum;
   wherein the composition has a viscosity of from 1,000,000 to 2,000,000 centipoise (cP) and a pH of from 5.0 to 7.5.
**Embodiment 12:** A denture adhesive composition comprising, consisting or consisting essentially of:
   (a) about 28% w/w of sodium carboxymethylcellulose;
   (b) about 8% w/w of a cross-linked polyacrylic acid homopolymer;
   (c) about 18% w/w of poly(methylvinylether/maleic acid) Sodium-Calcium Mixed Partial Salt;
   (d) about 17% w/w of an oil component;
   (e) about 29% of petrolatum; and optionally
   (f) at least one fragrance, colorant, preservative, surfactant or mixture thereof.
   wherein the composition has a viscosity of from 1,000,000 to 2,000,000 centipoise (cP) and a pH of from 5.0 to 7.5.
**Embodiment 13:** A denture adhesive composition comprising, consisting or consisting essentially of:
   (a) about 28% w/w of sodium carboxymethylcellulose;
   (b) about 6% w/w of a cross-linked polyacrylic acid homopolymer;
   (c) about 20% w/w of poly(methylvinylether/maleic acid) Sodium-Calcium Mixed Partial Salt;
   (d) about 17% w/w of an oil component;
   (e) about 29% of petrolatum; and optionally
   (f) at least one fragrance, colorant, preservative, surfactant or mixture thereof.
   wherein the composition has a viscosity of from 1,000,000 to 2,000,000 centipoise (cP) and a pH of from 5.0 to 7.5.
**Embodiment 14:** A denture adhesive composition comprising, consisting or consisting essentially of:
   (a) from 26-30% w/w of sodium carboxymethylcellulose;
   (b) from 6-9% w/w of a cross-linked polyacrylic acid homopolymer;
   (c) from 16-20% w/w of poly(methylvinylether/maleic acid) Sodium-Calcium Mixed Partial Salt;
   (d) from 17-23% w/w of an oil component;
   (e) from 23%-29% of petrolatum; and optionally
   (f) at least one fragrance, colorant, preservative, surfactant or mixture thereof.
**Embodiment 15:** The denture adhesive composition of Embodiment 14 wherein the composition has a pH of from 4.0 to 10, particularly a pH of from 5.0 to 8.0.
**Embodiment 16:** The denture adhesive composition of Embodiment 14 or 15 wherein the composition has a viscosity of from 500,000-2,500,000 centipoise (cP), particularly from 500,000-2,000,000 centipoise (cP) when measured within two months after preparation.
**Embodiment 17:** The denture adhesive composition of Embodiments 14-16 wherein the carboxymethylcellulose has a viscosity of about 1000 to about 2800 centipoise (cP) (measured in a 1% solution at 25°C).
**Embodiment 18:** The denture adhesive composition of Embodiment 17, wherein the carboxymethylcellulose consists of fine particles of which at least 80% pass through a 200 U.S. mesh sieve.
**Embodiment 19:** The denture adhesive composition of Embodiment 17 or 18 wherein the crosslinked polyacrylic acid homopolymer is an allyl pentaerythritol crosslinked homopolymer.
**Embodiment 20:** The denture adhesive composition of Embodiment 19 wherein the cross-linked polyacrylic acid homopolymer is a carbomer homopolymer Type A or Type B.
**Embodiment 21:** The denture adhesive composition of Embodiment 20 wherein the cross-linked polyacrylic acid homopolymer is Carbopol 971P NF and/or Carbopol 974P NF.
**Embodiment 22:** The denture adhesive composition of any one of Embodiments 18-21, wherein the alkyl vinyl ether-maleic acid copolymer or salt thereof is a poly(methylvinylether/maleic acid) Salt
**Embodiment 23:** The denture adhesive composition of Embodiment 22, wherein the alkyl vinyl ether-maleic copolymer or salt thereof is a poly(methylvinylether/maleic acid) Sodium-Calcium Mixed Partial Salt.
**Embodiment 24:** The denture adhesive composition of Embodiment 23 which comprises, consists or consists essentially of:
   (a) about 28% w/w of sodium carboxymethylcellulose;
   (b) about 8% w/w of a cross-linked polyacrylic acid homopolymer;
   (c) about 18% w/w of poly(methylvinylether/maleic acid) Sodium-Calcium Mixed Partial Salt;
   (d) about 21% w/w of an oil component;
   (e) about 25% of petrolatum; and optionally
   (f) at least one fragrance, colorant, preservative, surfactant or mixture thereof.
**Embodiment 25:** The denture adhesive composition of Embodiment 23 which comprises, consists or consists essentially of:
   (a) about 28% w/w of sodium carboxymethylcellulose;
   (b) about 6% w/w of a cross-linked polyacrylic acid homopolymer;
   (c) about 20% w/w of poly(methylvinylether/maleic acid) Sodium-Calcium Mixed Partial Salt;
   (d) about 21% w/w of an oil component;
   (e) about 25% of petrolatum; and optionally
   (f) at least one fragrance, colorant, preservative, surfactant or mixture thereof.
**Embodiment 26:** The denture adhesive composition of Embodiments 24 or 25, wherein the composition has a viscosity of from 1,000,000 to 2,000,000 centipoise (cP) and a pH of from 5.0 to 7.5.
**Embodiment 27:** The denture adhesive composition of any of Embodiments 1 to 26 wherein the composition is free of polyethylene glycol, sodium alginate and zinc.

In order that this invention may be better understood, the following examples are set forth. These examples are for purposes of illustration only and are not to be construed as limiting the scope of the invention in any manner.

### EXAMPLES

### Example 1: Denture adhesive compositions I

Six formulations were prepared using commercially available ingredients. The formulations were based on a commercially available product, POLIGRIP Cushion and Comfort denture adhesive cream (comprising: Cellulose gum, Paraffinum liquidum, Petrolatum, Carbomer, Silica, Cera Alba, Aroma and Limonene), adapted to incorporate a poly(methylvinylether/maleic acid) Sodium-Calcium Mixed Partial Salt component in order to increase hold performance:

| **Raw Material** | **1** | **2** | **3** | **4** | **5** | **6** |
|---|---|---|---|---|---|---|
| **Carbomer** | 9 | 8 | 7 | 6 | 5 | 4 |
| **AVE/MA^{∗}** | 4 | 6 | 8 | 10 | 12 | 14 |
| **CMC** | 40.82 | 39.82 | 38.82 | 37.82 | 36.82 | 35.82 |
| **Frescolat ML Crystalline** | 0.1 | 0.1 | 0.1 | 0.1 | 0.1 | 0.1 |
| **Mineral oil** | 26.26 | 26.26 | 26.26 | 26.26 | 26.26 | 26.26 |
| **Petrolatum** | 18.1 | 18.1 | 18.1 | 18.1 | 18.1 | 18.1 |
| **Beeswax** | 0.5 | 0.5 | 0.5 | 0.5 | 0.5 | 0.5 |
| **Cab-o-sil M5** | 1.1 | 1.1 | 1.1 | 1.1 | 1.1 | 1.1 |
| **Spearmint oil 50/50** | 0.075 | 0.075 | 0.075 | 0.075 | 0.075 | 0.075 |
| **Peppermint oil** | 0.045 | 0.045 | 0.045 | 0.045 | 0.045 | 0.045 |

| | | | | | | |
|---|---|---|---|---|---|---|
| ^{∗} poly(methylvinylether/maleic acid) Sodium-Calcium Mixed Partial Salt | | | | | | |

Briefly, the petrolatum component was melted, the mineral oil component was added followed by the melted beeswax with stirring. The carbomer, sodium carboxmethylcellulose, poly(methylvinylether/maleic acid) Sodium-Calcium mixed partial salt and Cab-o-sil M5 were then added in powder form with stirring. The composition was cooled and the flavour components (Frescolat, Spearmint oil and peppermint oil) added. After further mixing, the formulation was de-aerated.

To compare the adhesion profile of the denture adhesives, an Instron Texture Analyzer (5943) equipped with BioPuls Bath and 500 N load cell was used (Instron, UK). A quantity of adhesive was placed onto the test surface of the texture analyser, compressed to a uniform thickness of 1mm across the test surface and then taken through a number of cycles of compression/stretching. Following the compression/stretching cycles, the cross head was rapidly moved apart with speed of 6lbf/min and the maximum tensile force recorded. This was repeated at least 20 times for each sample.

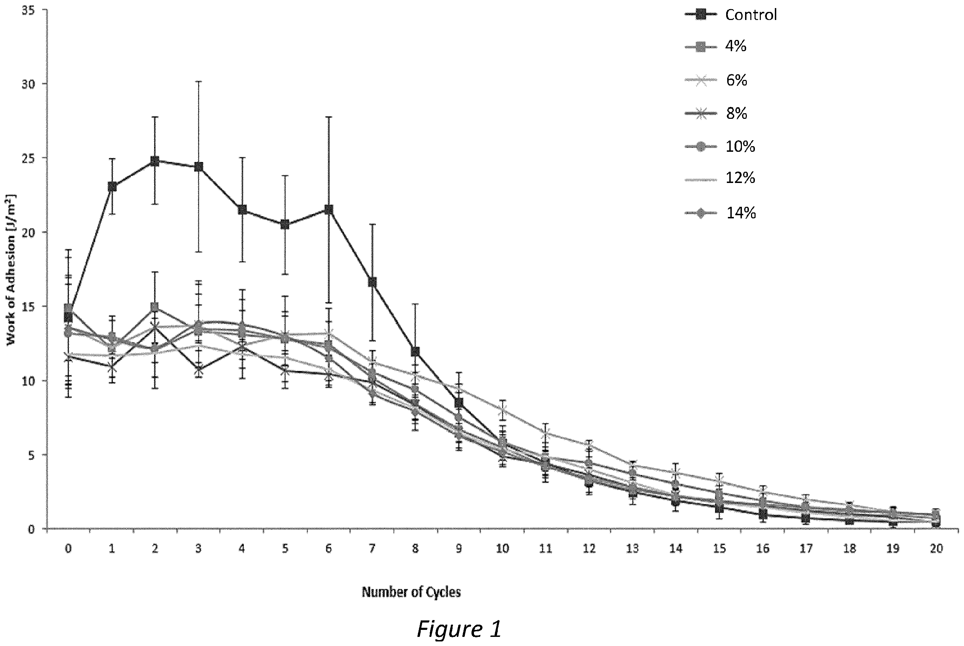

None of the formula options improved the hold profile sufficiently to match a control formulation (POLIGRIP Flavour Free denture adhesive cream, which contains Sodium-Calcium mixed partial salt but does not contain Carbopol) (Figure 1). Addition of 14% w/w or less poly(methylvinylether/maleic acid) Sodium-Calcium mixed partial salt to formulations was not sufficient to improve the adhesion profile.

### Example 2: Effect of carbomer on viscosity

Eight further formulations were prepared using commercially available ingredients. The formulations were based on the commercially available product, POLIGRIP Flavour Free denture adhesive cream (comprising Calcium/Sodium PVM/MA Copolymer, Petrolatum, Cellulose Gum and Paraffinum Liquidum), adapted to incorporate a carbomer component:

| **Raw Material** | **7** | **8** | **9** | **10** | **11** | **12** | **13** | **14** |
|---|---|---|---|---|---|---|---|---|
| **Carbomer** | 9 | 9 | 14 | 18 | 18 | 18 | 36 | 54 |
| **AVE/MA** | 36 | 9 | 20 | 0 | 18 | 36 | 9 | 0 |
| **CMC** | 9 | 36 | 20 | 36 | 18 | 0 | 9 | 0 |
| **Mineral oil** | 17 | 17 | 17 | 17 | 17 | 17 | 17 | 17 |
| **Petrolatum** | 29 | 29 | 29 | 29 | 29 | 29 | 29 | 29 |
| **Viscosity** | 1,812,000 | 1,862,000 | 3,124,000 | >4,000,000 | >4,000,000 | >4,000,000 | N/D | N/D |

| | | | | | | | | |
|---|---|---|---|---|---|---|---|---|
| ^{∗} poly(methylvinylether/maleic acid) Sodium-Calcium Mixed Partial Salt | | | | | | | | |

Formulations were prepared following similar processes described in International Patent Application publication number WO16091738. Briefly, the petrolatum component was melted and the mineral oil component added with stirring. Next, the carbomer, sodium carboxmethylcellulose, poly(methylvinylether/maleic acid) Sodium-Calcium mixed partial salt were added in powder form with stirring. The composition was cooled and de-aerated.

Initial viscosity of the formulations was measured using a controlled-stress rheometer (CarriMed CSL500) in dynamic mode with a cone-and-plate configuration in a constant strain mode with an angular velocity of 10 rad/s at 37°C. Five separate specimens were measured for each formulation.

The viscosity of formulations comprising at least 18% w/w of the carbomer component was very high (>4,000,000 cPs). Formulations 13 and 14, comprising 36% w/w and 54% w/w of the carbomer respectively, were too thick to enable proper formulation and testing.

Formulations comprising greater than 18% w/w of carbomer have viscosities that render them unsuitable for general use because they are difficult to formulate and/or would be difficult for a consumer to squeeze from a dispenser, such as a tube. Thus, it was determined that commercially desirable formulations should contain less than 18% w/w of carbomer.

### Example 3: Denture adhesive compositions II

Seven further formulations were prepared using commercially available ingredients. The formulations were based on a commercially available product, POLIGRIP Flavour Free denture adhesive cream (comprising Calcium/Sodium PVM/MA Copolymer, Petrolatum, Cellulose Gum and Paraffinum Liquidum), adapted to incorporate a carbomer, reduced AVE/MA level and varying quantities of CMC whilst maintaining an acceptable viscosity profile:

| **Raw Material** | **15** | **16** | **17** | **18** | **19** | **20** | **21** |
|---|---|---|---|---|---|---|---|
| **Carbomer** | 4 | 6 | 8 | 10 | 10 | 8 | 8 |
| **AVE/MA^{∗}** | 22 | 20 | 18 | 20 | 22 | 24 | 26 |

| **CMC** | 28 | 28 | 28 | 24 | 22 | 22 | 20 |
|---|---|---|---|---|---|---|---|
| **Mineral Oil** | 17 | 17 | 17 | 17 | 17 | 17 | 17 |
| **Petrolatum Viscosity** | 29 | 29 | 29 | 29 | 29 | 29 | 29 |
| | 1,224,000 | 1,544,000 | 1,382,000 | 1,730,000 | 1,698,000 | 1,422,000 | 1,484,000 |

| | | | | | | | |
|---|---|---|---|---|---|---|---|
| ^{∗} poly(methylvinylether/maleic acid) Sodium-Calcium Mixed Partial Salt | | | | | | | |

Formulations were prepared as previously described.

To compare the adhesion profile of the denture adhesives, an Instron Texture Analyzer (5943) equipped with BioPuls Bath and 500 N load cell was used (Instron, UK). A quantity of adhesive was placed onto the test surface of the texture analyser, compressed to a uniform thickness of 1mm across the test surface and then taken through a number of cycles of compression/stretching. Following the compression/stretching cycles, the cross head was rapidly moved apart with speed of 6lbf/min and the maximum tensile force recorded. This was repeated at least 20 times for each sample.

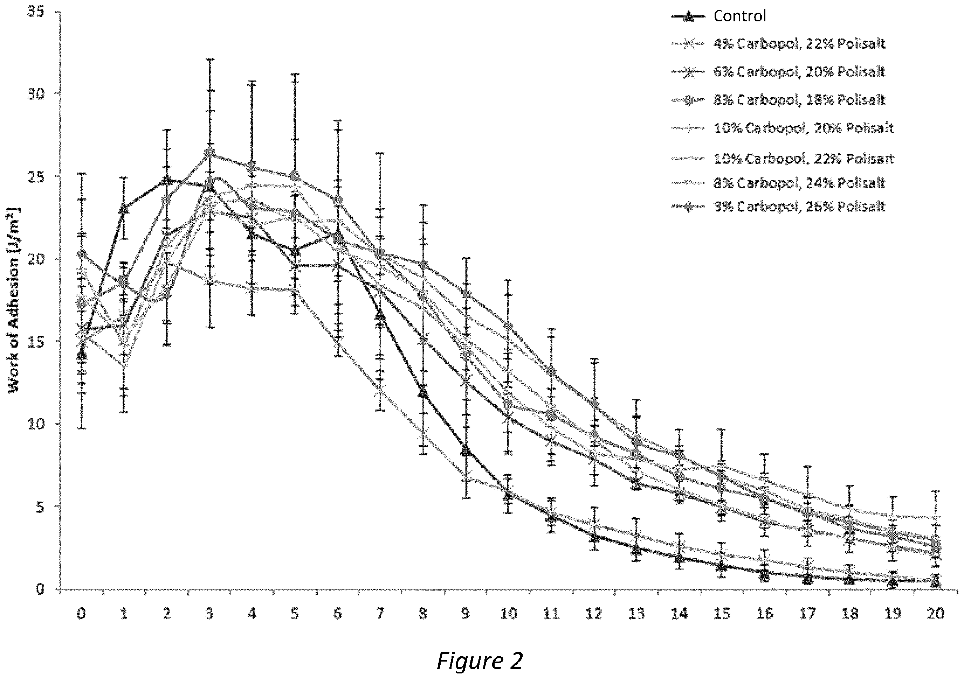

Results for the formulation comprising 4% Carbopol exhibited sub-optimal performance in comparison to the control. However, several of the formulations demonstrated hold performance more comparable or improved over the existing commercial formulation (Figure 2).

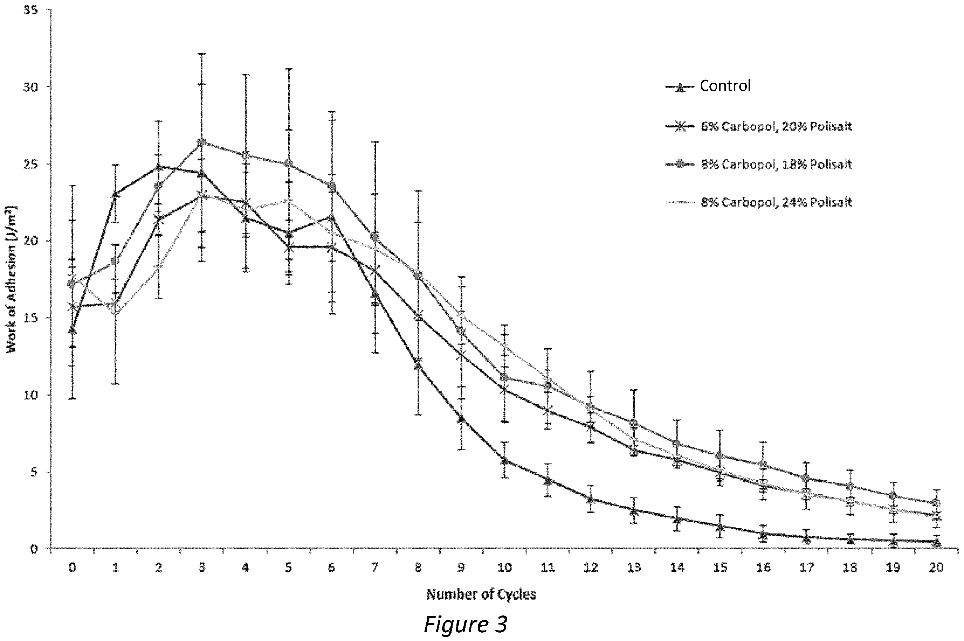

The formulation comprising 8% Carbopol and 18% poly(methylvinylether/maleic acid) Sodium-Calcium Mixed Partial Salt had the highest biopuls peak whilst the formulation comprising 6% Carbopol and 20% poly(methylvinylether/maleic acid) Sodium-Calcium Mixed Partial Salt exhibited similar performance to the commercial formulation. Both of these formulations exhibited higher work of adhesion scores in cycles 10 to 20 (Figure 3).

### Example 4: Mineral Oil and Petrolatum

Based on positive in-vitro data on hold and viscosity, formulations 16 (6% Carbopol/20% AVE/MA/28% CMC) and 17 (8% Carbopol/18% AVE/MA/28% CMC) were selected for further work.

The petrolatum and mineral oil levels were altered to determine impact of adhesion and viscosity.

| **Raw Material** | **16** | **22** | **23** | **24** | **25** |
|---|---|---|---|---|---|
| **Carbomer** | **6** | 6 | 6 | 6 | 8 |
| **AVE/MA^{∗}** | **20** | 20 | 20 | 20 | 18 |

| **CMC** | 28 | 28 | 28 | 28 | 28 |
|---|---|---|---|---|---|
| **Mineral Oil** | 17 | 19 | 21 | 23 | 21 |
| **Petrolatum Viscosity** | 29 | 27 | 25 | 23 | 25 |
| | 1,544,000 | 1,532,000 | 1,014,400 | 1,048,000 | |

Increasing the proportion of mineral oil whilst decreasing the proportion of petrolatum lowered the viscosity significantly. However, after an initial decrease in viscosity, increasing the proportion of mineral oil further, beyond 21% w/w had little effect.

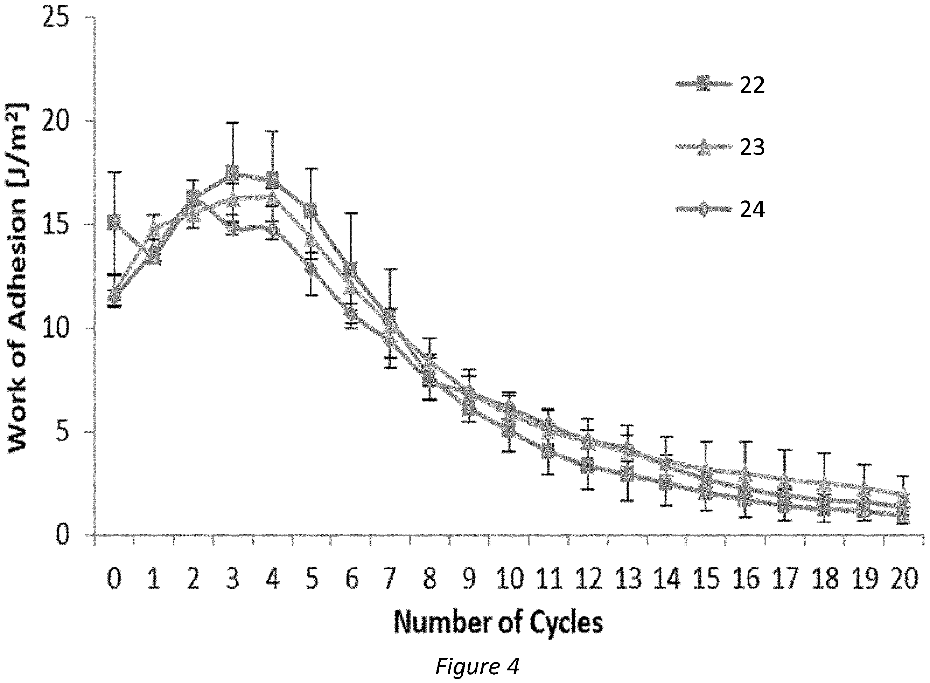

Changing the proportion of mineral oil and petrolatum did not impact the adhesion profiles of formulations 22, 23 and 24 (Figure 4).

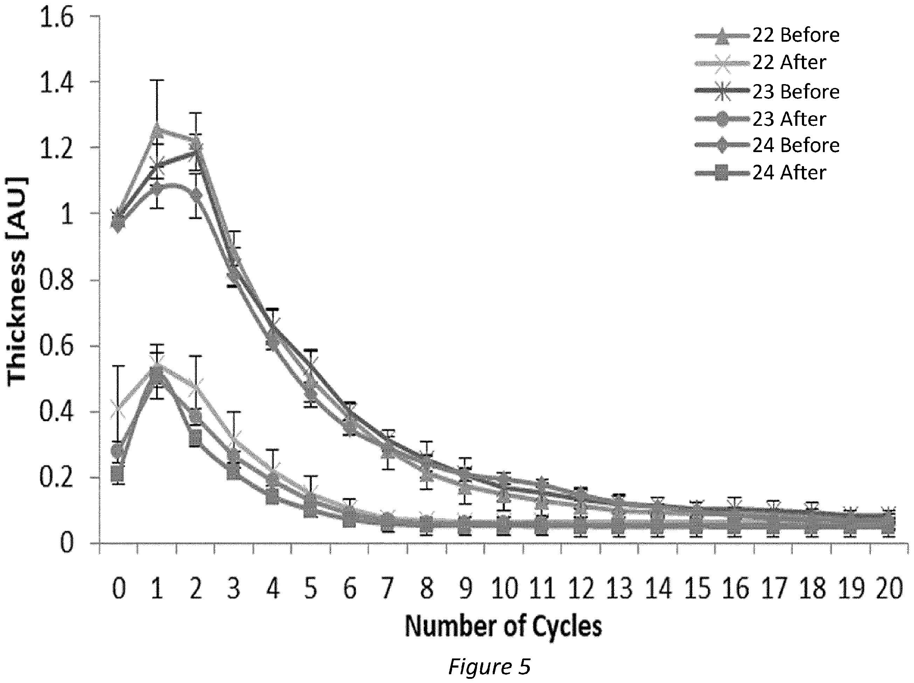

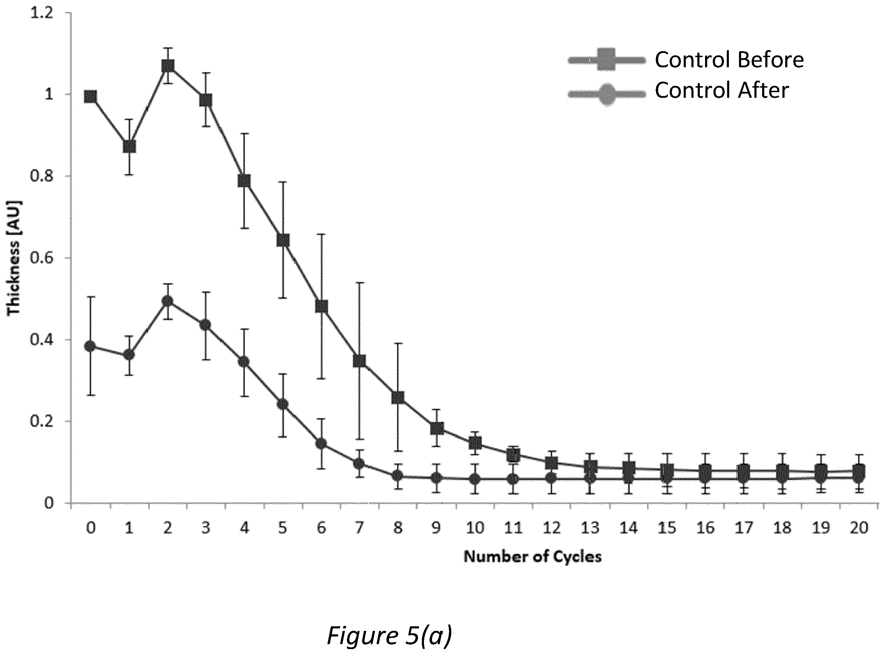

The adhesive layer thickness was also measured (Figure 5). Each of the formulations maintained a higher layer thickness than the control (Figure 5(a), potentially indicating improved cushioning effects.

The purpose of the above description is to illustrate some embodiments of the present invention without implying a limitation. It will be apparent to those skilled in the art that various modifications and variations may be made in the apparatus or procedure of the invention without departing from the scope or spirit of the invention.

## Claims

1. A denture adhesive composition comprising: (a) carboxymethylcellulose, (b) a cross-linked polyacrylic acid homopolymer and (c) an alkyl vinyl ether-maleic acid copolymer or salt thereof.

2. The denture adhesive composition of Claim 1 comprising: (a) from about 15 to about 30 percent by weight (%w/w) of carboxymethylcellulose, (b) from about 4 to about 15 percent by weight (%w/w) of the cross-linked polyacrylic acid homopolymer and (c) from about 15 to about 34 percent by weight (%w/w) of an alkyl vinyl ether-maleic acid copolymer or salt thereof.

3. The denture adhesive composition of Claim 1 or 2, further comprising (d) an oil component and/or (e) a petrolatum component.

4. The denture adhesive composition of Claim 1, 2 or 3, wherein the composition has (i) a viscosity of from 500,000-2,000,000 centipoise (cP) and/or (ii) a pH of from 4.0 to 10, particularly a pH of from 5.0 to 8.0.

5. The denture adhesive composition of Claim 3 or 4 comprising: (d) from about 17 to about 26 percent by weight (%w/w) of the oil component and (e) from about 20 to about 30 percent by weight (%w/w) of petrolatum.

6. The denture adhesive composition of Claim 5 wherein the carboxymethylcellulose has a viscosity of about 1000 to about 2800 centipoise (cP) (measured in a 1% solution at 25°C).

7. The denture adhesive composition of Claim 6, wherein the carboxymethylcellulose consists of fine particles of which at least 80% pass through a 200 U.S. mesh sieve.

8. The denture adhesive composition of Claim 6 or 7 wherein the carboxymethylcellulose is sodium carboxymethylcellulose.

9. The denture adhesive composition of any preceding claim which comprises from about 5 to about 10 percent by weight (%w/w) of the cross-linked polyacrylic acid homopolymer, particularly from about 6 to about 9 percent by weight (%w/w) of the cross-linked polyacrylic acid homopolymer.

10. The denture adhesive composition of Claim 8 wherein the cross-linked polyacrylic acid homopolymer is an allyl pentaerythritol crosslinked homopolymer.

11. The denture adhesive composition of Claim 10 wherein the cross-linked polyacrylic acid homopolymer is a carbomer homopolymer Type A or Type B.

12. The denture adhesive composition of Claim 11 wherein the cross-linked polyacrylic acid homopolymer is Carbopol 971P NF and/or Carbopol 974P NF.

13. The denture adhesive composition of any one of Claims 9 to 12, wherein the alkyl vinyl ether-maleic acid copolymer or salt thereof is a poly(methylvinylether/maleic acid) Salt

14. The denture adhesive composition of Claim 13, wherein the alkyl vinyl ether-maleic copolymer or salt thereof is a poly(methylvinylether/maleic acid) Sodium-Calcium Mixed Partial Salt.

15. The denture adhesive composition of any preceding claim which comprises or consists essentially of:
(a) from 26-30% w/w of sodium carboxymethylcellulose;
(b) from 6-9% w/w of a cross-linked polyacrylic acid homopolymer;
(c) from 16-20% w/w of poly(methylvinylether/maleic acid) Sodium-Calcium Mixed Partial Salt;
(d) from 17-23% w/w of an oil component;
(e) from 23%-29% of petrolatum; and optionally
(f) at least one fragrance, colorant, preservative, surfactant or mixture thereof.

16. The denture adhesive composition of Claim 15 which comprises, consists or consists essentially of:
(a) about 28% w/w of sodium carboxymethylcellulose;
(b) about 8% w/w of a cross-linked polyacrylic acid homopolymer;
(c) about 18% w/w of poly(methylvinylether/maleic acid) Sodium-Calcium Mixed Partial Salt;
(d) about 21% w/w of an oil component;
(e) about 25% of petrolatum; and optionally
(f) at least one fragrance, colorant, preservative, surfactant or mixture thereof.

17. The denture adhesive composition of Claim 15 which comprises, consists or consists essentially of:
(a) about 28% w/w of sodium carboxymethylcellulose;
(b) about 6% w/w of a cross-linked polyacrylic acid homopolymer;
(c) about 20% w/w of poly(methylvinylether/maleic acid) Sodium-Calcium Mixed Partial Salt;
(d) about 21% w/w of an oil component;
(e) about 25% of petrolatum; and optionally
(f) at least one fragrance, colorant, preservative, surfactant or mixture thereof.

18. The denture adhesive composition of Claims 15 to 17, wherein the composition has a viscosity of from 1,000,000 to 2,000,000 centipoise (cP) and a pH of from 5.0 to 7.5.

19. The denture adhesive composition of any of the claims 1 to 16 wherein the composition is free of polyethylene glycol, sodium alginate and zinc.
